# EUROPÄISCHE PATENTSCHRIFT

(11) **EP 0 923 560 B1**
(45) Veröffentlichungstag und Bekanntmachung des Hinweises auf die Patenterteilung: **08.01.2003**
(21) Anmeldenummer: 97937534.2
(22) Anmeldetag: 31.07.1997
(51) Int. Cl.: C07D 233/36, C07D 233/32, D06M 13/352

(54) **VERBRÜCKTE, GEGEBENENFALLS METHYLOLIERTE BIS-4,5-DIHYDROXY-IMIDAZOLIDIN-2-ONE, VERFAHREN ZU DEREN HERSTELLUNG UND VERWENDUNG DERSELBEN**
BRIDGED AND POSSIBLY METHYLOLIZED 4,5-BIS-DI-HIDROXY-IMIDAZOLIDIN-2-ONES, PROCESS FOR THEIR PRODUCTION AND USE THEREOF
BIS-4,5-DIHYDROXY-IMIDAZOLIDIN-2-ONES PONTEES ET EVENTUELLEMENT METHYLOLEES, LEURS PROCEDES DE PRODUCTION ET LEUR UTILISATION

(30) Priorität: 05.08.1996 DE 19631618
(43) Veröffentlichungstag der Anmeldung: 23.06.1999
(73) Patentinhaber: BASF AKTIENGESELLSCHAFT, 67056 Ludwigshafen (DE)
(72) Erfinder: HOIS, Pia, D-69488 Birkenau (DE); REICHERT, Jürgen, D-67117 Limburgerhof (DE)
(74) Vertreter: Isenbruck, Günter, Dr.
(86) Internationale Anmeldenummer: EP9704171
(87) Internationale Veröffentlichungsnummer: WO98005650

(56) Entgegenhaltungen:
- US-A- 3 890 095
- US-A- 4 298 747
- US-A- 4 306 872
- CHEMICAL ABSTRACTS, vol. 78, no. 8, 26.Februar 1973 Columbus, Ohio, US; abstract no. 44433h, H. NIEBERGALL ET. AL.: "Synthesis of dodecamethylenebis (dihydroxyethyleneurea)." Seite 31; Spalte 1; XP002046061 in der Anmeldung erwähnt & ANGEW. MAKROMOL. CHEM., Bd. 27, 1972, Seiten 113-28,

## Beschreibung

Die Erfindung betrifft verbrückte, gegebenenfalls methylolierte Bis-4,5-dihydroxy-imidazolidin-2-one, gemäß der nachstehenden allgemeinen Formel 1 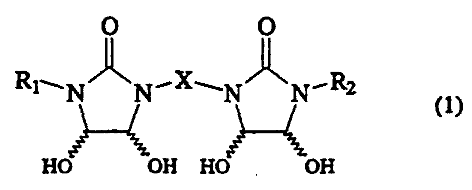
- wobei X =: (CH₂)ₙ mit n=2, 3, 4, 5, 7, 8, 9 oder 10, verzweigte oder cyclische Alkylketten, jeweils ohne oder mit Heteroatomen wie O, S, N, P in der Alkylkette,
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(-OCH₂C(CH₃)H)ₘ oder
CH₂CH₂CH₂(-OCH₂CH₂CH₂CH₂)ₘOCH₂CH₂CH₂ ist,
wobei m = 0 - 7,
und
R¹,R² = H und/oder CH₂OH sind,
4,5-Dihydroxy-1,3-dimethylolimidazolidin-2-one werden bei der Textilveredlung als formaldehydhaltige Textilvernetzer eingesetzt. Sie bewirken eine gute Knittererholung und Verringerung des Textilkrumpfes. Die Festigkeit des Gewebes wird jedoch stark beeinträchtigt. Außerdem führt der Einsatz dieser Substanzen auf Textilien zu erhöhten Formaldehydwerten, die von den meisten Anwendern aus ökotoxikologischen Gründen nicht mehr akzeptiert werden.

Auf den Gebieten der Lebensmittelchemie sind bestimmte Vernetzungsmittel bekannt, die dazu eingesetzt werden, die Gaspermeabilität von Zellglas, d.h. von Cellophan-Folien zu steuern. Die Verbindungen der allgemeinen Formel 1 mit X = (CH₂)₆ und (CH₂)₁₂ werden von H. Niebergall und H. Seitz [Die Angew. Makromolekulare Chem. 21 (1972) 41 bis 51; ibid 113 bis 128 und ibid 129 bis 142] beschrieben. Diese Verbindungen eignen sich - wie gesagt - zur Vernetzung von Zellglas, und insbesondere zur Steuerung der Gasdurchlässigkeit. Sie werden mittels eines zweistufigen Verfahrens hergestellt, bei dem unter Einsatz des entsprechenden verbrückten Harnstoffderivats und Glyoxal zunächst das entsprechende verbrückte Imidazolidin-2-on hergestellt wird. In einer zweiten Stufe wird das verbrückte Imidazolidin-2-on mit Formaldehyd zu der entsprechenden hydroxymethylierten Verbindung umgesetzt. Über den Einsatz dieser bekannten Stoffe bei der Textilveredlung ist in der Literatur nichts bekannt.

Dokument US 4,298,747 beschreibt Bis-4,5-dihydroxy-imidazolidin-2-one, die sich von den erfindungsgemäßen Substanzen der Formel 1 durch die Gruppen R¹ und R² unterscheiden, die entsprechend dem genannten Dokument Methylgruppen sind.

Die US 3,890,095 beschreibt die Verwendung von Monoimidazolidinonen, beispielsweise von Dimethyloldihydoxyethylenharnstoff (DMDHEU) in der Textilveredlung. Derartige Textilveredler sind zwar zur Reduzierung des Knitters geeignet, sie verschlechtern jedoch die Reißfestigkeit und führen insbesondere zu erhöhten Formaldehydwerten und sind daher aus ökotoxikologischen Gründen nicht mehr akzeptabel.

Die US 4,306,872 beschreibt Bis-imidazolidinone, die sich von den erfindungsgemäßen Bis-imidazolidinonen der Formel 1 durch die R¹- und R²-Gruppen unterscheiden: R¹ und R² haben in dieser Druckschrift die Bedeutung von Alkylradikalen mit 1 - 4 Kohlenstoffatomen.

Aus Dokument US 3,890,095 ist die Verwendung von Dimethyloldihydroxyethylenharnstoff (DMDHEU), d.h. einer Verbindung mit einem einzigen Imidazolidin-2-on-Heterozyklus, in der Textilveredlung, insbesondere zur Reduzierung des Knitters, bekannt. Wie in der Tabelle auf Seite 13 der vorliegenden Patentanmeldung aufgezeigt, weist jedoch die Ausrüstung mit DMDHEU Nachteile bezüglich der Reißfestigkeit des Gewebes sowie der Formaldehydbelastung auf (vergleiche Spalte 3, 3, sowie 5. bis 7. Zeile).

Demgegenüber werden erfindungsgemäß Verbindungen mit zwei verbrückten Imidazolidin-2-on-Heterozyklen zur Verfügung gestellt, die nicht nur einen wesentich geringeren Formaldehydgehalt aufweisen, sondern bei einer nahezu gleich guten Eignung zur Knitterreduzierung gleichzeitig die Reißfestigkeit der ausgerüsteten Textilien im Vergleich zu DMDHEU weniger beeinträchtigen. (Vergleiche hierzu die in der Tabelle auf Seite 13, Spalten 4 bis 9 aufgeführten Prüfergebnisse). Die erfindungsgemäßen Vernetzer zeigen somit in überraschender Weise einen doppelten positiven Effekt, wobei zwei Parameter für knitterfrei ausgerüstete Textilien, die Reißfestigkeit und der Formaldehydgehalt, gleichzeitig verbessert sind. Vorliegend wird somit nicht lediglich eine physikalische Größe derart optimiert, daß ein für den Verwendungszweck annehmbarer Kompromiß zwischen zwei gegenläufig von dieser Größe abhängigen Wirkungen erzielt wird. Zugleich soll bei ihrem Einsatz auf Textilien auch der Formaldehydwert nicht in ökologisch bedenklicher Weise erhöht werden.

Diese Aufgabe wird durch verbrückte, gegebenenfalls methylolierte Bis-4,5-dihydroxy-imidazolidin-2-one gemäß der Formel 1
- wobei X =: (CH₂)ₙ mit n=2, 3, 4, 5, 7, 8, 9 oder 10, verzweigte oder cyclische Alkylketten, jeweils ohne oder mit Heteroatomen wie O, S, N, P in der Alkylkette,
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(-OCH₂C(CH₃)H)ₘ oder
CH₂CH₂CH₂(-OCH₂CH₂CH₂CH₂)ₘOCH₂CH₂CH₂ ist,
wobei m = 0 - 7,
und
R¹,R² = H und/oder CH₂OH sind,
gelöst.

Gegenstand der Erfindung sind dementsprechend verbrückte, gegebenenfalls methylolierte Bis-4,5-dihydroxy-imidazolidin-2-one der Formel 1, ein zweistufiges Verfahren zu deren Herstellung, ein einstufiges Verfahren zur Herstellung der verbrückten, methylolierten Bis-4,5-dihydroxy-imidazolidin-2-one der
Formel 1 inklusive der Verbindung, bei der X = (CH₂)₆ ist, sowie allgemein die Verwendung dieser verbrückten, methylolierten Bis-4,5-dihydroxyimidazolidin-2-one inklusive derjenigen, bei der X = (CH₂)₆ ist, in der Textilveredlung.

Derartig behandelte Stoffe sind insbesondere in bezug auf ihre erhöhte Reißfestigkeit bevorzugt.

Überraschenderweise ist der Formaldehydgehalt auf dem Gewebe deutlich niedriger als erwartet.

Das Verfahren zur Herstellung der verbrückten, methylolierten Bis-4,5-dihydroxy-imidazolidin-2-one umfaßt erfindungsgemäß eine zweistufige oder eine einstufige Variante:

Die zweistufige Variante ist in den nachfolgend wiedergegebenen chemischen Reaktionsgleichungen beschrieben: 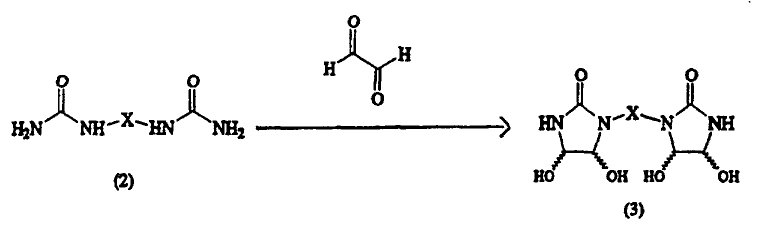 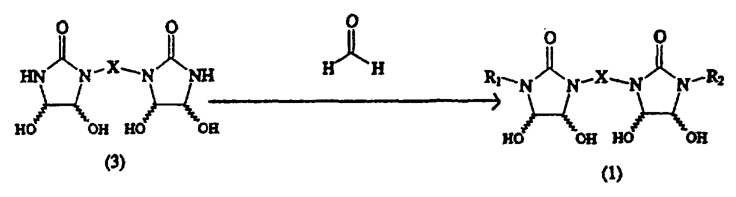 mit R₁, R₂ = H und/oder CH₂OH.

Ausgehend von den verbrückten Harnstoffen gemäß der vorstehend wiedergegebenen Formel (2) werden durch Umsetzung mit Glyoxal in Gegenwart von Basen, Säuren oder deren Salzen bei 30°C bis 70°C die Verbindungen der Formel (3) hergestellt. Bei dieser Umsetzung liegt der pH-Wert der Lösungen bevorzugterweise im Bereich von 4 bis 8. Aus den Verbindungen gemäß Formel (3) werden anschließend in einem zweiten Verfahrensschritt durch Umsetzung mit Formaldehyd im pH-Bereich von 4 bis 9 bei 30°C bis 70°C die gewünschten verbrückten, methylolierten Bis-4,5-dihydroxy-imidazolidin-2-one hergestellt.

Überraschenderweise können alternativ dazu die verbrückten, methylolierten Bis-4,5-dihydroxy-imidazolidin-2-one unter Einsatz der gleichen Reagenzien auch direkt in einem einstufigen Verfahren gemäß der nachfolgenden Gleichung 2 hergestellt werden.

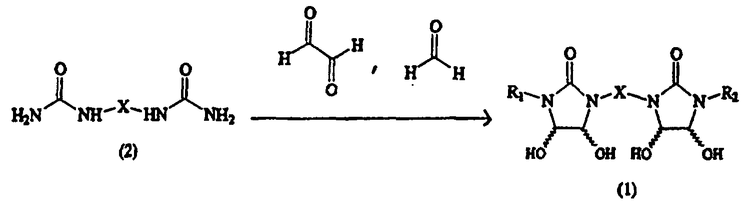

Bei der direkten, einstufigen Herstellung der verbrückten, methylolierten Bis-4,5-dihydroxy-imidazolidin-2-one werden die verbrückten Harnstoffe als Ausgangssubstanzen bevorzugt mit 1,7 bis 2,3, noch besser mit 1,8 bis 2,0 Moläquivalenten Glyoxal und 1,0 bis 2,3, bevorzugt 1,5 bis 2,0 Moläquivalenten Formaldehyd umgesetzt. Die Umsetzungen zu den verbrückten, methylolierten Bis-4,5-dihydroxy-imidazolidin-2-onen können mit unterschiedlich konzentrierten Glyoxal- und/oder Formaldehyd-Lösungen unter Verwendung von organischen oder anorganischen Säuren oder Basen oder Puffersubstanzen erfolgen. Der pH-Wert liegt dabei bevorzugt in einem Bereich von 4 bis 8. Die Temperatur beträgt geeigneterweise 30 bis 70°C. Als Puffersubstanzen kommen insbesondere Phosphat- oder Acetat-Puffer in Frage.

Die Erfindung wird nachfolgend durch Beispiele näher erläutert:

### Herstellungsbeispiele

### 1. Beispiel:

Ethylen-1,2-bis-(methylol-4,5-dihydroxy-ethylenharnstoff), X=CH₂CH₂

### A. einstufige Synthese

Zu 0,4 Mol Formaldehyd und 0,4 Mol Glyoxal wurden bei einem pH-Wert von ca. 5. 0,2 Mol Ethylenbisharnstoff gegeben. Im pH-Bereich von 6 bis 7 wurde mehrere Stunden bei 50°C gerührt. Als der freie Aldehydgehalt einen Umsatz von ca. 90 % anzeigte, wurde abfiltriert.
Auswaage: 106 g mit einem Wassergehalt von 52 %.
¹³C-NMR (Enantiomerengemisch)(D₂O)δ(ppm) = 40,8; 41,8 (NCH₂); 66,7; 66,8 (NCH₂OH); 79,5; 80,4; 81,4; 84,6; 86,3; 87,0; 87,6 (CHOH); 161,1; 161,2 (CO)

IR (cm⁻¹) = 3500-3200 (breit O-H); 2950 (m, C-H); 1697 (s, C=O); 1487 (s), 1326 (m), 1246 (s), 1030 (s)

Die starken Signale für Ethylendiharnstoff bei 1653 und 1604 cm⁻¹ waren nicht mehr vorhanden.

### B. zweistufige Synthese

Zu 0,4 Mol Glyoxal, das mit Natriumacetat auf pH-Wert = 5,4 eingestellt war, wurden 0,2 Mol Ethylenbisharnstoff gegeben. Nach 5 Stunden bei 40°C wurden 0,29 Mol Formaldehyd zugegeben, der pH-Wert auf 6 eingestellt und mehrere Stunden lang bei 50°C gerührt. Als der Aldehydgehalt einen Umsatz von ca. 90 % anzeigte, wurde die Reaktion beendet. Auswaage 98 g mit einem Wassergehalt von 52 %.

### 2. Beispiel:

Propylen-1,3-bis-(N-methylol-4,5-dihydroxy-ethylenharnstoff),
X = CH₂CH₂CH₂

### A. einstufige Synthese

Eine Lösung aus 0,4 Mol Formaldehyd mit 0,4 Mol Glyoxal wurde mit einem Phosphat-Puffer auf einen pH-Wert von ca. 5,5 eingestellt. Nach der Zugabe von 0,2 Mol Propylenbisharnstoff wurde auf 50°C erwärmt, der pH-Wert auf 6 bis 6,5 eingestellt und mehrere Stunden gerührt, bis der Umsatz größer als 90% war. Auswaage 110 g mit einem Wassergehalt von 46 %.

¹³C-NMR (Ennantiomerengemisch)(D₂O)δ(ppm) = 26,4; 26,6 (CH₂); 37,3; 37,6 (NCH₂); 62,5; 63,0 (NCH₂OH); 75,8; 77,5; 82,6; 84,3; 84,4 (cycl. CHOH); 156,7; 157,0 (CO)

IR(cm⁻¹)=3500-3200 (breit O-H);2944 (m, C-H); 1697 (s, C=O); 1487 (s), 1246 (s), 1030 (s)

Die starken Signale für Propylendiharnstoff bei 1647, 1598 und 1555 cm⁻¹ waren nicht mehr vorhanden.

### B. zweistufige Synthese

Zu 0,4 Mol Glyoxal, das mit Schwefelsäure auf pH-Wert von 1,4 eingestellt war, wurden 0,2 mol Propylenbisharnstoff gegeben. Gegebenenfalls durch Wasserzugabe wurde die Rührbarkeit erhöht. Nach 24 Stunden bei 40°C, wurden 0,29 Mol Formaldehyd zugegeben, der pH-Wert mit Natriumacetat auf 6 eingestellt und mehrere Stunden bei 50°C gerührt. Als der Aldehydgehalt ein Umsatz von ca. 90 % anzeigte, wurde abfiltriert. Auswaage 103 g mit einem Wassergehalt von 57 %.

### 3. Beispiel:

Hexamemylen-1,6-bis-(N-methylol-4,5-dihydroxy-ethylenharnstoff),
X = CH₂CH₂CH₂CH₂CH₂CH₂
Eine Lösung aus 0,58 Mol Formaldehyd und 0,6 Mol Glyoxal wurde mit Natronlauge auf einen pH-Wert von ca. 5,4 eingestellt und anschließend mit 50 ml Wasser verdünnt. Nach der Zugabe von 0,3 Mol Hexamethylenbisharnstoff wurde auf 55°C erwärmt, der pH-Wert auf 6 bis 7 eingestellt und mehrere Stunden gerührt, bis der Umsatz größer 90 % war; anschließend wurde abfiltriert. Auswaage 250 g mit einem Wassergehalt von 57 %. Durch Aufkonzentration bei ca. 20 Torr wurde ein Wassergehalt von 30 % eingestellt.

¹³C-NMR (Enantiomerengemisch) (D₂O) δ(ppm) = 28,2; 29,7; 42,5 (CH₂); 66,6 (NCH₂OH); 79,3; 80,4; 86,0; 87,0 (CHOH); 161,0 (CO)

IR(cm⁻¹)=3500-3200(breit O-H); 2938 (m,C-H); 2864 (m, C-H), 1697 (s, C=O); 1493 (s), 1246 (s), 1030 (s)

Die starken Signale für Hexamethylendiharnstoff 1654, 1598 und 1561 cm⁻¹ waren nicht mehr vorhanden.

### 4. Beispiel:

Cyclohexamethylen-1,4-bis-(N-methylol-4,5-dihydroxy-ethylenharnstoff),
X = cyclohexyl
Eine Lösung aus 0,4 Mol Formaldehyd und 0,4 Mol Glyoxal wurde mit Natronlauge auf einem pH-Wert von ca. 5,4 eingestellt. Nach der Zugabe von 0,2 Mol Cyclohexyl-bisharnstoff wurde langsam auf 50°C erwärmt, der pH-Wert auf 6 bis 7 eingestellt und mehrere Stunden gerührt, bis der Umsatz an Aldehyd größer 90 % war, anschließend wurde abfiltriert. Auswaage 112 g einer Suspension mit einem Wassergehalt von 41 %.

IR(cm⁻¹)=3500-3200(breit O-H); 2950 (m,C-H); 2858 (m,C-H), 1690 (s, C=O), 1690(s, C=O); 1487(s), 1265 (m), 1079 (m) 1030 (m).

Die starken Signale für Hexamethylendiharnstoff 1660, 1598 und 1549cm⁻¹ waren nicht mehr vorhanden.

### 5. Beispiel:

2,2-Dimethyl-propylen-1,3-bis-(N-methylol-4,5-dihydroxy-ethylenharnstoff),
X = CH₂C(CH₃)₂CH₂
Herstellung analog Beispiel 4.

¹³C-NMR (Enantiomerengemisch) (D₂O) δ(ppm) = 25,7; 26,0; 26,5 (CH₃); 40,9; 40,9; 41,2 (C); 50,4; 50,7; 51,0; 51,2 (NCH₂); 66.3; 66,8 (CH₂OH); 79,3; 82,3; 85,6; 88,2 (CHOH); 162,0; 162; 2 (CO)

IR(cm⁻¹)=3500-3200(breit O-H); 2963 (m,CH); 1684 (s,C=O); 1486 (s), 1258 (m), 1036 (m).

Die starken Signale für 2,2-Dimethyl-propylendiharnstoff 1604, 1579 und 1561 cm⁻¹ waren nicht mehr vorhanden.

### 6. Beispiel:

2-Hydroxy-propylen-1,3-bis-(N-methylol-4,5-dihydroxy-ethylenharnstoff),
X = CH₂CH(OH)CH₂
Herstellung analog Beispiel 4 aber mit 0,05 % Phosphat.

¹³C-NMR (Enantiomerengemisch) (DMSO) δ(ppm) = 43,8; 43,9; 44,1 (NCH₂); 62,6; 62,9; 63,0 (CH₂OH); 66,5; 66,7; 67,3; 67,5; 68,6 (CHOH); 75,8; 77,9; 78,0; 78,5; 81,8; 82,5; 84,6; 85,3; 85,4 (cycl.CHOH); 156.7; 156,8; 156,9 (CO)

IR(cm⁻¹) =3500-3200 (breit O-H); 2944 (m,C-H); 1691 (s,C=O); 1493 (s), 1252 (m), 1024 (m).
Die starken Signale für 2-Hydroxy-propylendiharnstoff 1629 und 1579 cm⁻¹ waren nicht mehr vorhanden.

### 7. Beispiel:

3,6-Dioxa-octan-1,8-bis-(N-methylol-4,5-dihydroxy-ethylenharnstoff),
X = CH₂CH₂OCH₂CH₂OCH₂CH₂
Herstellung analog Beispiel 4.

### 8. Beispiel:

4-Oxa-heptan-1,7-bis-(N-methylol-4,5-dihydroxy-ethylenharnstoff)
X = CH₂CH₂CH₂OCH₂CH₂CH₂
Zu 0,4 Mol Formaldehyd und 0,4 Mol Glyoxal wurden bei einem pH-Wert von ca. 5,2 0,2 mol 4-Oxa-heptan-1,7-bisharnstoff gegeben. Die Reaktionslösung wurde mit 1,2 Mol Wasser verdünnt. Im pH-Bereich von 6,3 bis 6,5 wurde 24 Stunden bei 50°C gerührt. Das abfiltrierte Produkt hatte einen Wassergehalt von 50,5 %.

¹³C-NMR (Enantiomerengemisch) (D₂O) δ(ppm) = 30,0; 30,2 (CH₂); 40,0; 40,1 (NCH₂); 66,5; 66,6; 66,7 (CH₂OH); 70,7; 70,8 (CH₂OR); 79,4; 80,8; 86,1; 87,2 (cycl.CHOH); 161,1; 161,3 (CO)

IR(cm⁻¹)=3500-3200(breit O-H); 2956 (m, C-H); 2870 (m, C-H); 1690 (s, C=O); 1493 (s), 1252 (m), 1024 (m).

Die starken Signale für 3,6-Dioxa-octan-1,8-diharnstoff 1654, 1604 und 1570 cm⁻¹ waren nicht mehr vorhanden.

### 9. Beispiel:

4,7-Dioxa-decan-1,10-(N-methylol-4,5-dihydroxy-ethylenharnstoff)
X = CH₂CH₂CH₂OCH₂CH₂OCH₂CH₂CH₂
Herstellung analog Beispiel 4.

IR(cm⁻¹)=3500-3200 (breit O-H); 2950 (m, C-H); 2876 (m, C-H); 1678 (s, C=O); 1493 (s), 1246 (m), 1085 (m), 1023 (m).

Die starken Signale für 4,7-Dioxa-decen-1,10-diharnstoff bei 1654 und 1567 cm⁻¹ waren nicht mehr vorhanden.

### 10. Beispiel:

4,9-Dioxa-dodecan-1,12-(N-methylol-4,5-dihydroxy-ethylenharnstoff)
X = CH₂CH₂CH₂-OCH₂CH₂CH₂CH₂-OCH₂CH₂CH₂
Herstellung analog Beispiel 4.

¹³C-NMR (Enantiomerengemisch) (D₂O) δ(ppm) = 27,9 (CH₂); 29,8; 30,0 (CH₂); 39,7; 39,9 (CH₂); 66,6; 66,4; (OCH₂); 70,2; 70,4 (OCH₂); 72,9; (OCH₂); 79,1; 80,5; 85,8; 87,0 (cycl. CHOH); 160,7; 160,9 (CO)

IR(cm⁻¹)=3500-3200 (breit O-H); 2950 (m, C-H); 2870 (m, C-H); 1697 (s, C=O); 1499 (s), 1258 (m), 1116 (m), 1023(m).

Die starken Signale für 4,9-Dioxa-dodecan-1,12-diharnstoff bei 1666 und 1567 cm⁻¹ waren nicht mehr vorhanden.

### Anwendungstechnische Prüfung:

### a) Behandlung der Testgewebe:

Von den Produkten wurde eine 4%ige Lösung (bez. 100% Feststoff) hergestellt, die als Katalysator noch 1,2% Magnesiumchlorid krist. enthielt. Das Testgewebe (Baumwolle) wurde in einem Foulard mit diesen Lösungen imprägniert und die Flottenaufnahme auf 75% begrenzt. Anschließend wurde bei 120°C auf 6 bis 8% Restfeuchte getrocknet. Die Kondensation erfolgte in 4 min bei 150°C.

In der nachfolgenden Tabelle beinhalten die Spalten 2 und 3 Vergleichsbeispiele und die weiteren Spalten 4 - 9 Beispiele nach der Erfindung.

Die zweite, mit "0" bezeichnete Spalte beinhaltet die Prüfergebnisse für ein Gewebe ohne Zusatz eines Textilveredlers. Der in Spalte 3 aufgeführte Textilveredler DMDHEU ist die in der Textilveredlung bekannte Verbindung mit einem einzigen Imidazolidin-2-on-Heterozyklus, und zwar Dimethyloldihydroxyethylenharnstoff.

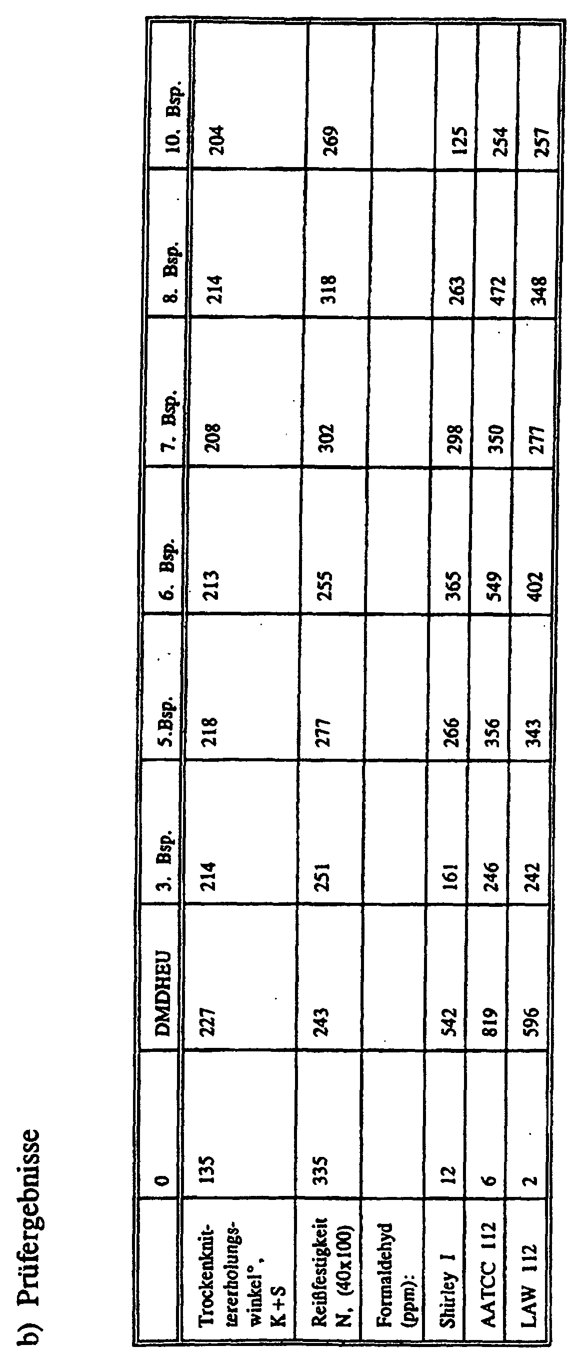

## Patentansprüche

1. Verbrückte, gegebenenfallls methylolierte Bis-4,5-dihydroxy-imidazolidin-2-one gemäß der nachfolgenden Formel 1 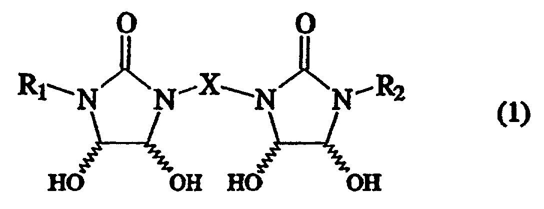
wobei X = (CH₂)ₙ mit n=2, 3, 4, 5, 7, 8, 9 oder 10, verzweigte oder cyclische Alkylketten, jeweils ohne oder mit Heteroatomen wie O, S, N, P in der Alkylkette,
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(-OCH₂C(CH₃)H)ₘ oder
CH₂CH₂CH₂(-OCH₂CH₂CH₂CH₂)ₘOCH₂CH₂CH₂ ist,
wobei m = 0 - 7,
und
R¹,R² = H und/oder CH₂OH sind.

2. Verfahren zur Herstellung der verbrückten, gegebenenfalls methylolierten Bis-4,5-dihydroxy-imidazolidin-2-one nach Anspruch 1, wobei ein verbrücktes Harnstoffderivat gemäß der nachfolgenden Formel 2 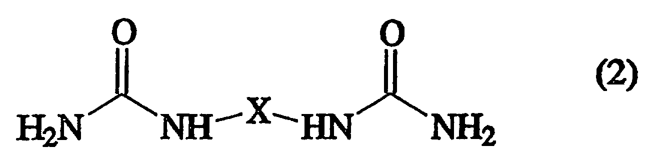
wobei X = (CH₂)ₙ mit n=2, 3, 4, 5, 7, 8, 9 oder 10, verzweigte oder cyclische Alkylketten, jeweils ohne oder mit Heteroatomen wie O, S, N, P in der Alkylkette,
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(-OCH₂C(CH₃)H)ₘ oder
CH₂CH₂CH₂(-OCH₂CH₂CH₂CH₂)ₘOCH₂CH₂CH₂ ist,
wobei m = 0 - 7,
in einem zweistufigen Verfahren zunächst mit Glyoxal bei 30°C bis 70°C reagieren gelassen wird und das so erhaltene Produkt anschließend mit Formaldehyd bei einer Temperatur von 30 bis 70°C umgesetzt wird.

3. Verfahren nach Anspruch 2, wobei die Umsetzung mit dem Glyoxal in einem pH-Bereich von 4 bis 8 und die Umsetzung mit Formaldehyd in einem pH-Bereich von 4 bis 9 erfolgt.

4. Verfahren zur Herstellung von verbrückten, gegebenenfalls methylolierten Bis-4,5-dihydroxy-imidazolidin-2-onen, gemäß Formel 1, wobei ein verbrücktes Harnstoffderivat gemäß Formel 2 in einem einstufigen Verfahren mit Glyoxal und Formaldehyd bei 30°C bis 70°C und einem pH-Wert von 4 bis 9 reagieren gelassen wird.

5. Verfahren nach Anspruch 4, wobei die verbrückten Harnstoffderivate gemäß Formel 2 mit 1,7 bis 2,3 Moläquivalenten Glyoxal und 1 bis 2,3 Moläquivalenten Formaldehyd umgesetzt werden.

6. Verwendung der verbrückten, gegebenenfalls methylolierten Bis-4,5-dihydroxy-imidazolidin-2-one gemäß der nachfolgenden Formel 1a 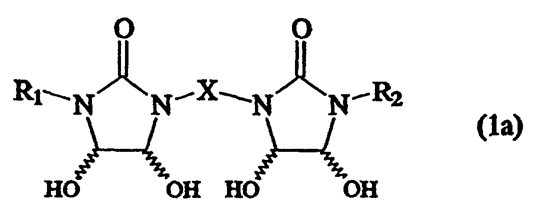
wobei X = (CH₂)ₙ mit n=2, 3, 4, 5, 6, 7, 8, 9 oder 10, verzweigte oder cyclische Alkylketten, jeweils ohne oder mit Heteroatomen wie O, S, N, P in der Alkylkette,
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(-OCH₂C(CH₃)H)ₘ oder
CH₂CH₂CH₂(-OCH₂CH₂CH₂CH₂)ₘOCH₂CH₂CH₂ ist,
wobei m = 0 - 7,
und
R¹,R² = H und/oder CH₂OH sind,
bei der Textilveredlung.

## Claims

1. Bridged, optionally methylolated bis-4,5-dihydroxyimidazolidin-2-ones conforming to the formula 1 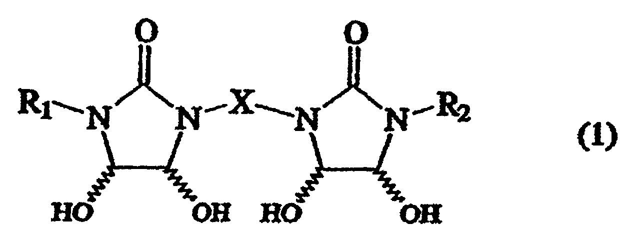
where X = (CH₂)ₙ where n=2, 3, 4, 5, 7, 8, 9 or 10, branched or cyclic alkyl, each with or without hetero atoms such as O, S, N, P in the alkyl chain,
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(-OCH₂C(CH₃)H)ₘ or
CH₂CH₂CH₂(-OCH₂CH₂CH₂CH₂)ₘOCH₂CH₂CH₂,
where m = 0 - 7,
and
R¹, R² = H and/or CH₂OH.

2. A process for preparing the bridged, optionally methylolated bis-4,5-dihydroxyimidazolidin-2-ones of claim 1, which comprises in a first step reacting a bridged urea derivative of the formula 2 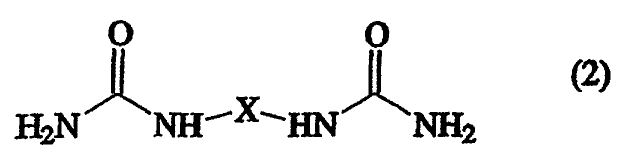
where X = (CH₂)ₙ where n=2, 3, 4, 5, 7, 8, 9 or 10, branched or cyclic alkyl, each with or without hetero atoms such as O, S, N, P in the alkyl chain,
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(-OCH₂C(CH₃)H)ₘ or
CH₂CH₂CH₂(-OCH₂CH₂CH₂CH₂)ₘOCH₂CH₂CH₂,
where m = 0 - 7,
with glyoxal at from 30°C to 70°C and then, in a second step, reacting the resulting product with formaldehyde at from 30 to 70°C.

3. A process as claimed in claim 2, wherein the reaction with the glyoxal takes place at from pH 4 to 8 and the reaction with formaldehyde takes place at from pH 4 to 9.

4. A process for preparing bridged, optionally methylolated bis-4,5-dihydroxyimidazolidin-2-ones conforming to the formula 1, wherein a bridged area derivative of the formula 2 is reacted with glyoxal and formaldehyde at from 30°C to 70°C and at from pH 4 to 9 in a single step.

5. A process as claimed in claim 4, wherein the bridged urea derivatives of the formula 2 are reacted with from 1.7 to 2.3 mol equivalents of glyoxal and from 1 to 2.3 mol equivalents of formaldehyde.

6. The use of the bridged, optionally methylolated bis-4,5-dihydroxyimidazolidin-2-ones conforming to the formula 1a 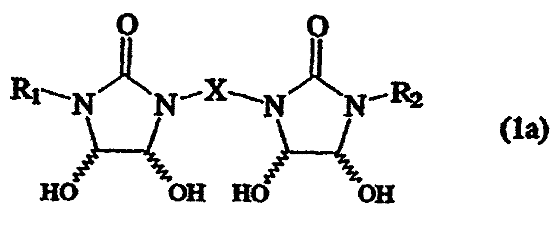
where X = (CH₂)ₙ where n=2, 3, 4, 5, 6, 7, 8, 9 or 10, branched or cyclic alkyl, each with or without hetero atoms such as O, S, N, P in the alkyl chain,
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(-OCH₂C(CH₃)H)ₘ or
CH₂CH₂CH₂(-OCH₂CH₂CH₂CH₂)ₘOCH₂CH₂CH₂,
where m = 0 - 7,
and
R¹, R² = H and/or CH₂OH,
in textile finishing.

## Revendications

1. Bis-4,5-dihydroxy-imidazolidin-2-ones pontées et éventuellement méthylolées selon la formule 1 suivante 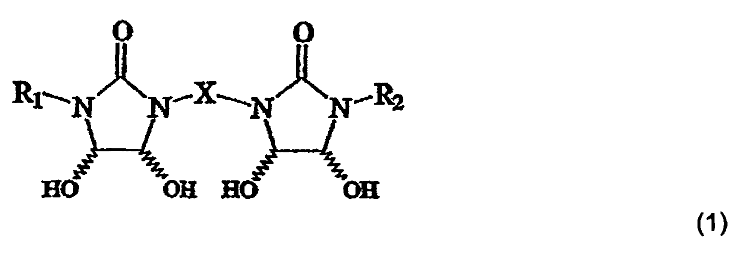
dans laquelle X = (CH₂)ₙ avec n = 2, 3, 4, 5, 7, 8, 9 ou 10 chaînes alkyles ramifiées ou cycliques, chacune sans ou avec des hétéroatomes comme O, S, N, P dans la chaîne alkyle, est
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(OCH₂C(CH₃)H)ₘ ou
CH₂CH₂CH₂(-OCH₂CH₂CH₂CH₂)ₘOCH₂CH₂CH₂,
où m = 0 - 7
et
R¹,R² = H et/ou sont CH₂OH.

2. Procédé de production de bis-4,5-dihydroxy-imidazolidin-2-ones pontées et éventuellement méthylolées selon la revendication 1, dans lequel un dérivé ponté de l'urée selon la formule 2 suivante : 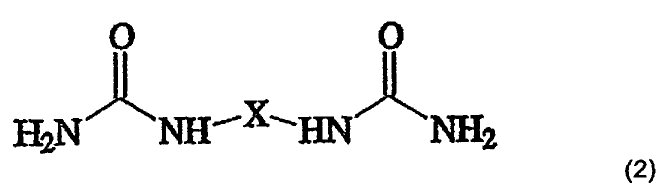
dans laquelle X = (CH₂)ₙ avec n = 2, 3, 4, 5, 7, 8, 9 ou 10 chaînes alkyles ramifiées ou cycliques, chacune sans ou avec des hétéroatomes comme O, S, N, P dans la chaîne alkyle, est
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(OCH₂C(CH₃)H)ₘ ou
CH₂CH₂CH₂(-OCH₂CH₂CH₂CH₂)ₘOCH₂CH₂CH₂,
où m = 0 - 7
est, dans un procédé en deux phases, tout d'abord laissé à réagir avec du glyoxal à 30°C jusqu'à 70°C et le produit ainsi obtenu est ensuite transformé avec du formaldéhyde à une température de 30 à 70°C.

3. Procédé selon la revendication 2, dans lequel la transformation avec le glyoxal est effectuée dans une gamme de pH de 4 à 8 et la transformation avec du formaldéhyde dans une gamme de pH de 4 à 9.

4. Procédé de production de bis-4,5-dihydroxy-imidazolidin-2-ones pontées et éventuellement méthylolées selon la formule 1, dans laquelle un dérivé ponté de l'urée selon la formule 2 est laissé à réagir, dans un procédé à une phase, avec du glyoxal et du formaldéhyde à 30 jusqu'à 70°C et à une valeur de pH de 4 à 9.

5. Procédé selon la revendication 4, dans lequel les dérivés pontés de l'urée selon la formule 2 sont transformés avec 1,7 à 2,3 équivalents molaires de glyoxal et 1 à 2,3 équivalents molaires de formaldéhyde.

6. Utilisation de bis-4,5-dihydroxy-imidazolidin-2-ones pontées et éventuellement méthylolées selon la formule 1a 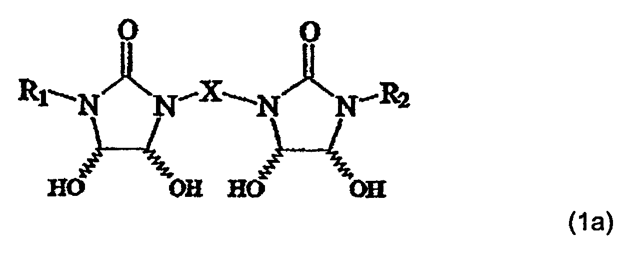
dans laquelle X = (CH₂)ₙ avec n = 2, 3, 4, 5, 6, 7, 8, 9 ou 10 chaînes alkyles ramifiées ou cycliques, chacune sans ou avec des hétéroatomes comme O, S, N, P dans la chaîne alkyle, est
CH₂CH₂(-OCH₂CH₂)ₘ,
CH₂CH₂CH₂(-OCH₂CH₂CH₂)ₘ,
CH₂CH₂CH₂(-OCH₂CH₂)ₘOCH₂CH₂CH₂),
CH₂CH₂(-OCH₂CH₂CH₂)ₘOCH₂CH₂,
CH₂CH₂CH₂CH₂(-OCH₂CH₂CH₂CH₂)ₘ,
CH₂C(CH₃)H(OCH₂C(CH₃)H)ₘ ou
CH₂CH₂CH₂(-OCH₂CH₂CH₂CH₂)ₘOCH₂CH₂CH₂,
où m = 0 - 7
et
R¹,R² = H et/ou sont CH₂OH.
dans l'ennoblissement des textiles.
